# EUROPÄISCHE PATENTSCHRIFT

(11) **EP 3 701 987 B1**
(45) Veröffentlichungstag und Bekanntmachung des Hinweises auf die Patenterteilung: **09.06.2021**
(21) Anmeldenummer: 19159923.2
(22) Anmeldetag: 28.02.2019
(51) Int. Cl.: A61M 15/00

(54) **PHARMAZEUTISCHER SPENDER, INSBESONDERE INHALATOR**
PHARMACEUTICAL DISPENSER, IN PARTICULAR INHALER
DISTRIBUTEUR PHARMACEUTIQUE, EN PARTICULIER INHALATEUR

(43) Veröffentlichungstag der Anmeldung: 02.09.2020
(73) Patentinhaber: Aptar Radolfzell GmbH, 78315 Radolfzell (DE)
(72) Erfinder: DU BOISBAUDRY, Guillame, 76230 Bois-Guillaume (FR); KÖRNER, Joachim, 88690 Uhldingen-Mühlhofen (DE)
(74) Vertreter: Patentanwaltskanzlei Cartagena

(56) Entgegenhaltungen:
- WO-A1-95/07723
- WO-A1-2019/025738
- GB-A- 2 398 065
- GB-A- 2 470 188
- US-A- 6 138 669
- US-A1- 2003 075 171
- US-A1- 2007 272 764

## Beschreibung

### ANWENDUNGSGEBIET UND STAND DER TECHNIK

Die Erfindung betrifft den Bereich der pharmazeutischen Spender, insbesondere jedoch nicht ausschließlich den Bereich der Dosierinhalatoren (Metered Dose Inhaler).

Bei Spendern für pharmazeutische Medien und insbesondere pharmazeutische Flüssigkeiten kann es von Relevanz sein, dass eine Betätigung oder der tatsächliche Flüssigkeitsaustrag erfasst werden. Eine hierauf basierende Auswertung kann im einfachsten Falle der Zählung bereits erfolgter Betätigungen und/oder Austragvorgängen dienen. Weitere Arten der Auswertung umfassen jedoch auch die Speicherung von Austragvorgängen für spätere Auswertung und/oder die Übermittlung von Daten zum Austrag an einen Server, so dass der Hersteller des Spenders und/oder eine mit der Verschreibung des Medikaments im Zusammenhang stehende Instanz wie beispielsweise der Arzt auf solche Daten zugreifen kann.

Spender, die mit elektronischen Erfassungsmitteln zur Erfassung von Austrägen versehen sind, sind vergleichsweise teuer. Es ist daher bereits vorgeschlagen worden, Spender modular auszugestalten, sodass Komponenten wiederverwendbar sind. Problematisch ist, dass Module mit elektronischen Komponenten, die an bestehende Flüssigkeitsspender nachträglich angefügt werden, Nachteile in Hinblick auf die Messgenauigkeit aufweisen, da sie meist nur mittelbar mit dem Austrag in Verbindung stehende Größen erfassen und auswerten. Spender, die mit elektronischen Erfassungsmitteln zur Erfassung von Austrägen versehen sind, sind beispielsweise aus den Patentschriften GB2470188A, WO2019025738A1 und WO9507723A1 bekannt.

### AUFGABE UND LÖSUNG

Aufgabe der Erfindung ist es, ein modulares System zur Verfügung zu stellen, welches die Nachteile des Standes der Technik vermindert.

Erfindungsgemäß wird ein pharmazeutischer Spender zum Austrag einer pharmazeutischen Flüssigkeit vorgeschlagen. Hierbei handelt es sich beispielsweise um einen Inhalator zum Austrag eines Medikaments in zerstäubter Form.

Der Spender weist einen Flüssigkeitsspeicher und/oder einen Aufnahmeraum zur Aufnahme eines wechselbaren Flüssigkeitsspeichers auf. Er weist weiterhin einen Auslass auf, durch den die Flüssigkeit aus dem Flüssigkeitsspeicher in eine Umgebung abgegeben oder durch den von einem Benutzer inhaliert werden kann. Ein Flüssigkeitskanal verbindet den Flüssigkeitsspeicher mit dem Auslass und gestattet damit die Ausgabe der pharmazeutischen Flüssigkeit.

Zur elektronischen Erkennung und Verarbeitung von Austragvorgängen weist der Spender eine Auswertungselektronik auf. Diese verfügt über mindestens einen Flüssigkeitssensor zur Erfassung einer durch das Strömen von Flüssigkeit vom Flüssigkeitsspeicher zum Auslass beeinflussten Größe.

Der erfindungsgemäße Spender ist modular aufgebaut und weist mindestens ein Hauptmodul und ein Auswertungsmodul auf, die zur lösbaren Kopplung aneinander ausgebildet sind. Das Hauptmodul umfasst dabei einerseits den Flüssigkeitsspeicher oder den zu seiner Aufnahme vorgesehenen Aufnahmeraum sowie andererseits den Auslass, im Falle eines Inhalator üblicherweise ein Mundstück. Das Auswertungsmodul umfasst die Auswertungselektronik sowie insbesondere auch zumindest einen Teilabschnitt des Flüssigkeitskanals sowie den an diesem Teilabschnitt vorgesehenen Flüssigkeitssensor.

Ein erfindungsgemäßer Spender weist somit einen modularen Aufbau auf, bei dem ein Hauptmodul den Auslass und den Flüssigkeitsspeicher oder dessen Aufnahmeraum bildet. Dieses Hauptmodul ist vorzugsweise frei von elektronischen Komponenten. Insbesondere vorzugsweise weist es die typische L-Form auf, die bei MDI-Spendern üblich ist und bei der ein Mundstück als Auslass am unteren Ende angewinkelt gegenüber einem vertikalen Aufnahmebereich für eine Containereinheit vorgesehen ist.

Das Hauptmodul ist vorzugsweise sehr einfach ausgestaltet und daher preisgünstig herstellbar. Es kann somit als regelmäßig austauschbares Moduls vorgesehen sein. Das Hauptmodul kann als Ganzes einstückig ausgebildet sein, um die Herstellungskosten gering zu halten. Es kann jedoch auch aus mehreren Bauteilen bestehen. Insbesondere kann der Auslass bzw. das Mundstück als gegenüber dem Aufnahmeraum für den Flüssigkeitsspeicher getrenntes Bauteil ausgebildet sein.

Das Auswertungsmodul weist die Auswertungselektronik auf. Diese umfasst neben dem eigentlichen Flüssigkeitssensor vorzugsweise einen Prozessor zur Auswertung der erfassten Daten sowie ggf. weitere Komponenten, die dem Zweck der Informationsdarstellung dienen (Display, LEDs, Lautsprecher) und/oder die dem Zweck der Speicherung und vorzugsweise drahtlosen Weitergabe der erfassten Daten an einen externen Server dienen (Bluetooth-Modul, WLAN-Modul, 3G-, 4G- oder5G-Modul).

Darüber hinaus umfasst das Auswertungsmodul auch mindestens zumindest einen Teilabschnitt des Flüssigkeitskanals, also mindestens einen Flächenbereich, der bei bestimmungsgemäßer Verwendung des Spenders in Flüssigkeitskontakt gelangt. Hierdurch wird die Messgenauigkeit verbessert, da anderenfalls die Sensorik bei Kopplung von Auswertungsmodul und Hauptmodul in den Bereich des einzig durch das Hauptmodul gebildeten Flüssigkeitskanals gebracht werden müsste. Dies allerdings würde dazu führen, dass die Position des Sensors variabler wäre und die Messergebnisse somit weniger aussagekräftig. Zudem ist der Teilabschnitt des Flüssigkeitskanals, der Teil des abkoppelbaren Auswertungsmoduls ist, auch leichter zu reinigen.

Grundsätzlich sind Gestaltungen möglich, bei denen das Auswertungsmodul vollständig im Hauptmodul aufgenommen sein kann, so dass das Auswertungsmodul im gekoppelten Zustand nicht oder nur durch die Oberfläche des Hauptmoduls hindurch sichtbar ist. Vorzugsweise wird jedoch eine äußere Oberfläche des Spenders von dem Hauptmodul und dem Auswertungsmodul gemeinsam gebildet. Jener Teil der Oberfläche des Spenders, der vom Auswertungsmodul gebildet wird, kann dann insbesondere auch genutzt werden, um hier ein Display und/oder LEDs, einen Lautsprecher oder Bedienelemente unterzubringen.

Das Hauptmodul und das Auswertungsmodul verfügen vorzugsweise über zusammenwirkende Kopplungseinrichtungen, die zur werkzeuglosen Koppelung und Entkoppelung ausgebildet sind. Die zusammenwirkenden Kopplungseinrichtungen sind vorzugsweise zur werkzeuglosen und zerstörungsfreien Entkopplung vorgesehen. Der Endkunde soll bestimmungsgemäß zur Entkoppelung und Koppelung in der Lage sein, sei es zum Austausch eines der Module oder zur Reinigung der Module.

Die Kopplungseinrichtungen sind insbesondere vorzugsweise zur Verschnappung miteinander ausgebildet. Im Falle solcher Verschnappungen sind diese vorzugsweise werkzeuglos und leicht zu lösen. Insbesondere kann bei einer Gestaltung, bei der das Auswertungsmodul nicht vollständig im Hauptmodul aufgenommen ist und mit einer Außenschale selbst einen Teil der äußeren Oberfläche des Spenders bildet, diese Außenschale voneinander beabstandet einen ersten und einen zweiten Teil der Kopplungseinrichtung auf, die zur Kopplung mit korrespondierenden Strukturen am Hauptmodul vorgesehen ist. Die beiden Teile der Kopplungseinrichtung auf Seite des Auswertungsmoduls können durch elastisches Verformen der Außenschale in einen entkoppelungsfähigen Zustand gebracht werden. Diese Außenschale ist zu diesem Zweck vorzugsweise ausreichend leicht manuell verformbar.

Wie bereits erläutert, bildet das Auswertungsmodul zumindest einen Teilabschnitt des Flüssigkeitskanals und weist in diesem Bereich eine für die Durchströmung mit Flüssigkeit geeignete Sensorik auf. Dabei kann es sich beim Teilabschnitt im einfachsten Falle um einen Flächenabschnitt handeln, der in eine korrespondierende Aussparung eines im Übrigen durch das Hauptmodul gebildeten Kanalabschnitts eingefügt wird.

Der Teilabschnitt des Flüssigkeitskanals, der Teil des Auswertungsmoduls ist, wird jedoch vorzugsweise durch einem umlaufend geschlossenen Kanalabschnitt gebildet, der selbst über einen Kanaleingang und einen Kanalausgang verfügt. Hierdurch wird die Abdichtung deutlich vereinfacht. Im Idealfalle wird der Flüssigkeitskanal ausgehend von einem Kopplungselement zur Ankopplung des Flüssigkeitsspeichers bis hin zu einem dem Auslass vorgeschalteten Flüssigkeitsauslass vollständig vom Auswertungsmodul gebildet, um die Kopplung des Auswertungsmoduls an das Hauptmodul möglichst problemlos zu gestalten.

Zur Erzielung einer kostengünstigen Bauweise und insbesondere zur Vermeidung vermeidbarer Schwierigkeiten die Abdichtung betreffend kann das Auswertungsmodul ein einstückiges Kanalbauteil aufweisen, welches sowohl den Kanaleingang als auch den Kanalausgang bildet.

Der Kanaleingang ist dabei vorzugsweise zur Aufnahme eines Auslassstutzens vorgesehen. Ein solcher Auslassstutzen kann Teil einer Containereinheit sein, die auch den Flüssigkeitsspeicher umfasst und zur Aufnahme im Aufnahmeraum vorgesehen ist. Solche verlagerbaren Auslassstutzen zur Öffnung des Flüssigkeitsspeichers wirken üblicherweise auf ein im Container integriertes Auslassventil. Der Kanaleingang ist bei einer solchen Gestaltung gleichzeitig Lagerfläche, gegen die der Auslassstutzen zum Zwecke der Ventilöffnung gedrückt wird.

Im Kanalabschnitt zwischen Kanaleingang und Kanalausgang ist der mindestens eine Flüssigkeitssensor bzw. eine Flüssigkeitssensorik umfassend mehrere Einzelsensoren vorgesehen. Dabei kann zum einen der Sensor an der direkten Kanalverbindung zwischen Kanaleingang und Kanalausgang vorgesehen sein. Bevorzugt ist je nach Art des Sensors jedoch eine Gestaltung, bei der zwischen dem Kanaleingang und dem Kanalausgang ein Abzweig in eine Messkammer vorgesehen ist. Auch diese kann durch das einstückige Kanalbauteil gebildet sein. Zwischen dem Abzweig und der Messkammer selbst ist eine Verjüngung vorgesehen, von der aus sich der Kanalabschnitt in Richtung der Messkammer und in Richtung des Abzweigs aufweitet. Die räumliche Trennung der Erfassung einer Flüssigkeitsgröße wie insbesondere des Flüssigkeitsdrucks vom unmittelbaren Verbindungskanal zwischen Kanaleinlass und Kanalauslass kann die Messung und Auswertung vereinfachen. In Hinblick auf die Messkammer und deren Vorteile wird auch auf die DE 102013214601 B3 verwiesen.

Der Teilabschnitt des Flüssigkeitskanals im Auswertungsmodul umfasst vorzugsweise einen auslenkbaren Wandungsabschnitt, vorzugsweise in Art einer Membran, der in Abhängigkeit des Flüssigkeitsdrucks ausgelenkt wird. Im Falle einer Gestaltung mit Messkammer ist dieser Wandungsabschnitt in der Messkammer angeordnet.

Dem Wandungsabschnitt ist der Flüssigkeitssensor in Form eines Drucksensors oder eines Schalters zugeordnet. Der Sensor bzw. der Schalter sind durch die Auslenkung des Wandungsabschnitts beeinflussbar bzw. schaltbar. In Hinblick auf eine Membran als Wandungsabschnitt und deren Vorteile wird auch auf die DE 102014206350 B3 verwiesen.

Neben Sensoren, die im Bereich eines solchen Wandungsabschnittes dessen Auslenkung erfassen, können Flüssigkeitssensoren im Auswertungsmodul auch auf viele andere Arten ausgestaltet sein. So kann ein Erschütterungssensor oder ein Mikrofon im Bereich des Flüssigkeitskanals die durchströmende Flüssigkeit wahrnehmen. Auch ein Temperatorsensor oder mehrere Temperatursensoren können verwendet werden, insbesondere als Teil eines thermischen Strömungssensors, der durch zweimalige Messung stromaufwärts und stromabwärts einer Wärmequelle den Flüssigkeitsstrom erfasst. Auch mittels induktiver und kapazitiver Sensoren kann der Flüssigkeitsstrom erfasst werden, insbesondere auch im Kontext des genannten Wandungsabschnitts, dessen Auslenkung sich derart erfassen lässt. Weiterhin kommt auch ein Differenzdrucksensor zur Erfassung des Flüssigkeitsstroms in Frage.

Vorzugsweise ist der Spender in Art eines typischen MDI-Spenders gestaltet. Dies bedeutet, dass er einen Aufnahmeraum zur Aufnahme eines wechselbaren Flüssigkeitsspeichers aufweist. Er weist weiterhin einen wechselbaren Flüssigkeitsspeicher auf, der Teil einer wechselbaren Containereinheit ist, welche in den genannten Aufnahmeraum in einer Container-Einschubrichtung eingeschoben ist. Hierbei ist der Flüssigkeitsspeicher vorzugsweise zum überwiegenden Teil in den Aufnahmeraum eingeschoben, also so, dass mindestens 50% des Flüssigkeitsspeichers im Aufnahmeraum aufgenommen sind.

Der Auslass ist bei einem solchen MDI-Spender als Mundstück ausgebildet, wobei dieses Mundstück in einer Erstreckungsrichtung ausgerichtet ist, die mit der Container-Einschubrichtung einen Winkel zwischen 45° und 135° einschließt.

Bei einem solchen MDI-Spender ist vorzugsweise vorgesehen, dass das Hauptmodul des Spenders ein Gehäuse bildet, welches auf einer dem Mundstück gegenüberliegenden Seite eine Einschuböffnung aufweist, in die das Auswertungsmodul in einer Kopplungsrichtung, also in Richtung des Mundstücks, eingeschoben ist.

Bei einer bevorzugten Gestaltung ist vorgesehen, dass das Hauptmodul und das Auswertungsmodul eine Führungsschienenstruktur aufweisen, die sich in Kopplungsrichtung erstreckt. Diese Führungsschienenstruktur weist mindestens auf Seite des Hauptmoduls oder des Auswertungsmoduls beidseitig einer Einschuböffnung je eine längliche Schiene auf, die je einen Gleitabschnitt oder je einen anderweitigen Gleiter auf Seiten des anderen Moduls gleitbeweglich aufnimmt.

Neben der hierdurch vereinfachten Koppelbarkeit der Module dient diese Führungsschienenstruktur auch der Aufnahme einer Betätigungskraft, wenn die Containereinheit des MDI-Spenders niedergedrückt wird.

Vorzugsweise weist die Führungsschienenstruktur mindestens eine Führungsnut auf, die an einem Ende eine Anschlagsfläche aufweist. Damit erfolgt die Kopplung der Module mit einer besonders klar definierten Endlage, was insbesondere dann von Vorteil ist, wenn die Containereinheit beim Einschub in das Hauptmodul unmittelbar an den Kanaleingang am Auswertungsmodul ankoppeln soll.

Die Kopplungsrichtung ist vorzugsweise gegenüber der Container-Einschubrichtung angewinkelt, vorzugsweise in einem Winkel zwischen 60° und 120° und insbesondere vorzugsweise in einem rechten Winkel.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 bis 4 zeigen den erfindungsgemäßen Spender und dessen Zusammenbau durch Zusammenfügen eines Hauptmoduls und eines Auswertungsmoduls sowie der Einfügung einer Containereinheit.
Fig. 5 und 6 zeigen das Hauptmodul und das Auswertungsmodul in einer geschnittenen Seitenansicht im zerlegten und montierten Zustand.
Fig. 7 bis 9 zeigen das Auswertungsmodul und seine Bestandteile im Detail.
Fig. 10 zeigt das Hauptmodul von seiner hinteren Seite aus.
Fig. 11 zeigt den Gesamtspender in geschnittener Ansicht.
Fig. 12 zeigt den Flüssigkeitspfad des Spenders beim Austrag und der Erfassung des Austrags.

### DETAILLIERTE BESCHREIBUNG DER AUSFÜHRUNGSBEISPIELE

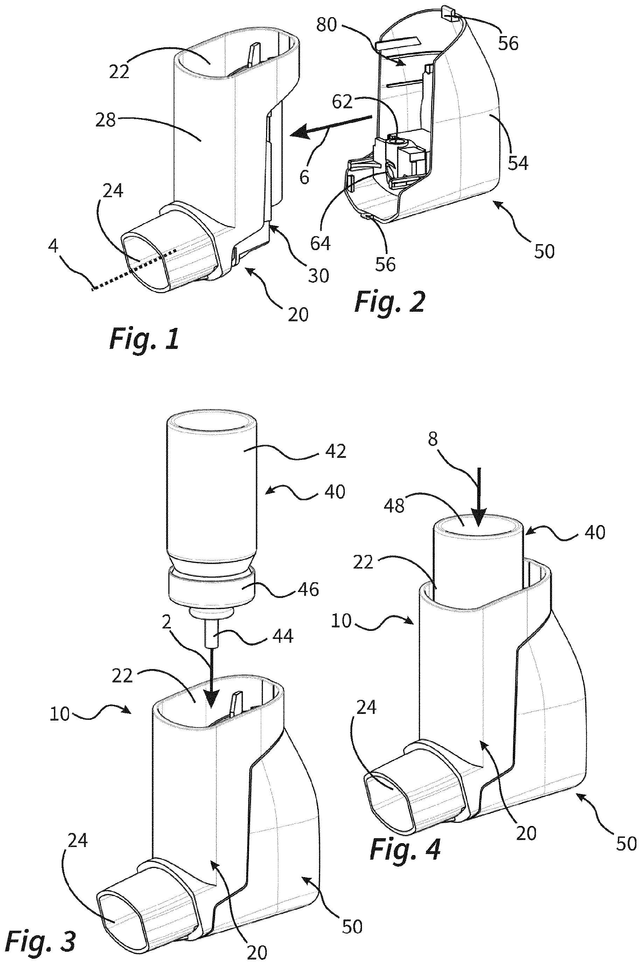

Fig. 1 bis 4 zeigen einen erfindungsgemäßen Spender 10 und dessen Zusammenbau.

Bei dem Spender 10 handelt es sich um einen sogenannten MDI-Spender (Metered Dose Inhaler). Wie bei solchen Spendern gattungstypisch vorgesehen, weist der Spender 10 eine in etwa L-förmige Formgebung auf, wobei er im vertikalen Abschnitt über einen nach oben offenen Aufnahmeraum 22 verfügt, in dem im betriebsfertigen Zustand eine Containereinheit 40 angeordnet ist, die einen Flüssigkeitsspeicher 42 und darin die auszutragende Flüssigkeit enthält.

Durch Niederdrücken dieser Containereinheit in Betätigungsrichtung 8 (siehe Fig. 4) wird eine Flüssigkeitsdosis aus der Containereinheit 40 abgegeben und durch einen weiteren noch beschriebenen Flüssigkeitskanal 14 zu einem Auslass 24 in Form eines Mundstücks gefördert. Wie aus den Fig. 1 und 2 ersichtlich ist, verfügt der Spender 10 über zwei wesentliche Module, nämlich ein Hauptmodul 20 und ein Auswertungsmodul 50. Das Hauptmodul 20 ist vorliegend einstückig ausgebildet. Sein Gehäuse 28 bildet den nach oben offenen Aufnahmeraum 22 für die Containereinheit 40 und stellt weiterhin den Auslass 24 in Form eines Mundstücks zur Verfügung.

Das Auswertungsmodul 50, dessen Komponenten im Weiteren noch erläutert werden, wird in einer Kopplungsrichtung 6 von der dem Auslass 24 gegenüberliegenden Seite an das Hauptmodul 20 angekoppelt. Hierfür weist das Hauptmodul 20 eine Einschuböffnung 30 auf, die als Durchbrechung des Gehäuses 28 ausgestaltet ist. Das Auswertungsmodul 50 verfügt über eine Außenschale 54, die am unteren und am oberen Ende jeweils mit zungenartigen Kopplungseinrichtungen 56 versehen ist.

Wie Fig. 3 verdeutlicht, erfolgt das Einsetzen der Containereinheit 40 nach vorheriger Kopplung des Hauptmoduls 20 und des Auswertungsmoduls 50. Das Einsetzen erfolgt in einer Containereinschubrichtung2. Die bereits erwähnte Fig. 4 zeigt den durch Kopplung der Module 20, 50 und Einsetzen der Containereinheit 40 fertiggestellten und betriebsfertigen Spender 10.

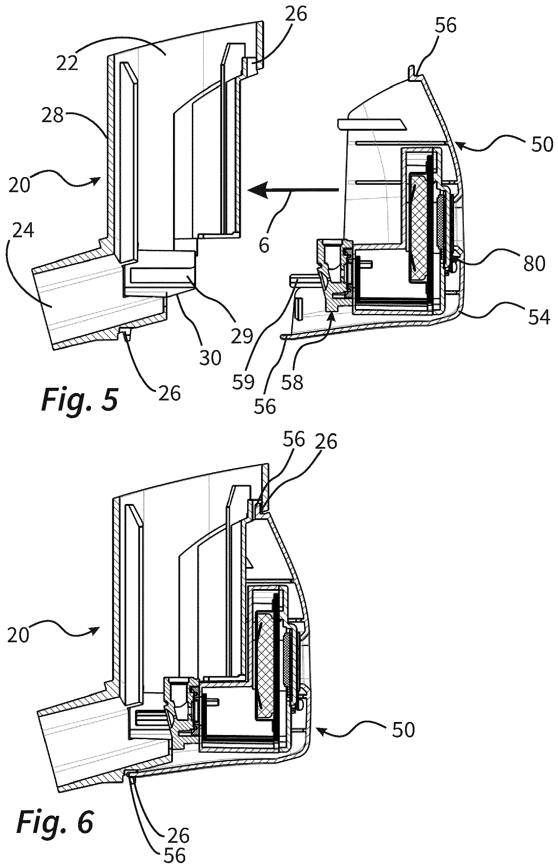

Die Fig. 5 und 6 zeigen den Kopplungsvorgang nochmals in geschnittener Darstellung. Es ist zu ersehen, dass korrespondierend mit den zungenartigen Kopplungseinrichtungen 56 am Auswertungsmodul 50 auch Kopplungseinrichtungen 26 am Hauptmodul 20 vorgesehen sind. Es handelt sich um Schlitze, in die die zungenartigen Kopplungseinrichtungen 56 des Auswertungsmoduls 50 eingefügt werden. Um insbesondere die Kopplung im Bereich der oberen Kopplungseinrichtung 56 und des korrespondierenden Schlitzes 26 zu bewerkstelligen, ist die Außenschale 54 des Auswertungsmoduls 50 aus einem leicht verformbaren Kunststoff gefertigt, so dass sie in sich ausreichend verformbar ist, um das Einrücken der jeweiligen Kopplungseinrichtungen 56 auf Seiten des Auswertungsmoduls 50 in die korrespondierenden Kopplungseinrichtungen 26 des Hauptmoduls 20 zu gestatten, um den gekoppelten Zustand der Fig. 6 zu erzielen.

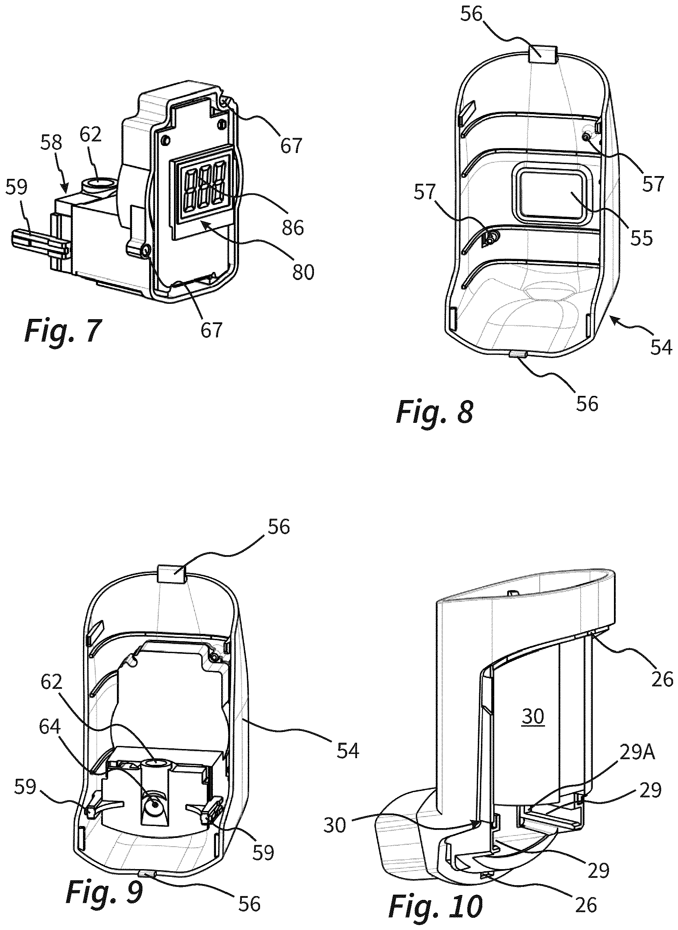

Die Fig. 7 bis 9 verdeutlichen den Aufbau des Auswertungsmoduls 50, insbesondere im Hinblick auf die für die Kopplung erforderlichen Elemente. Fig. 10 zeigt das Hauptmodul 20 und dessen korrespondierende Elemente.

Das Auswertungsmodul 50 ist nach außen abgeschlossen durch die in Fig. 8 separat dargestellte Außenschale 54. An dieser sind die zungenartigen Kopplungseinrichtungen 56 einstückig angeformt. Weiterhin ist eine Aussparung 55 für ein Display in der Außenschale 54 vorgesehen. Innenseitig verfügt die Außenschale 54 weiterhin über zwei Kopplungsstifte 57, zu denen korrespondierend eine in Fig. 7 dargestellte Inneneinheit des Auswertungsmoduls 50 Kopplungslöcher 67 aufweist. Die Inneneinheit, die alle elektronischen Elemente in sich vereint, kann somit fest mit der Außenschale 54 verkoppelt werden. Diese Kopplung ist bestimmungsgemäß nicht zur Trennung für den Endbenutzer vorgesehen.

Die Inneneinheit der Fig. 7 weist neben dem Display 86 weitere in Fig. 8 nicht im Detail dargestellte elektronische Komponenten auf. Darüber hinaus verfügt sie über ein Kanalbauteil 58, welches einen im Weiteren noch beschriebenen Kanaleingang 62 umfasst und welches zusätzlich in Kopplungsrichtung 6 erstreckte Stifte 59 aufweist, die Teil einer Führungsschienenstruktur sind. Korrespondierend zu diesen sind an dem in Fig. 10 dargestellten Hauptmodul 20 Führungsnuten 29 vorgesehen, an deren distalem Ende eine Anschlagsfläche 29a angeordnet ist. Zum Zwecke der Kopplung der Module wird somit das fertig montierte Auswertungsmodul 50 entsprechend der Fig. 9 mit den Stiften 59 in die Führungsnuten 29 des Hauptmoduls eingeschoben. Gegen Ende dieses Kopplungsvorgangs wird die Außenschale 54 durch den Benutzer leicht verformt, so dass die zungenartigen Kopplungseinrichtungen 56 in die korrespondierenden Kopplungseinrichtungen 26 des Hauptmoduls 20 einrücken können.

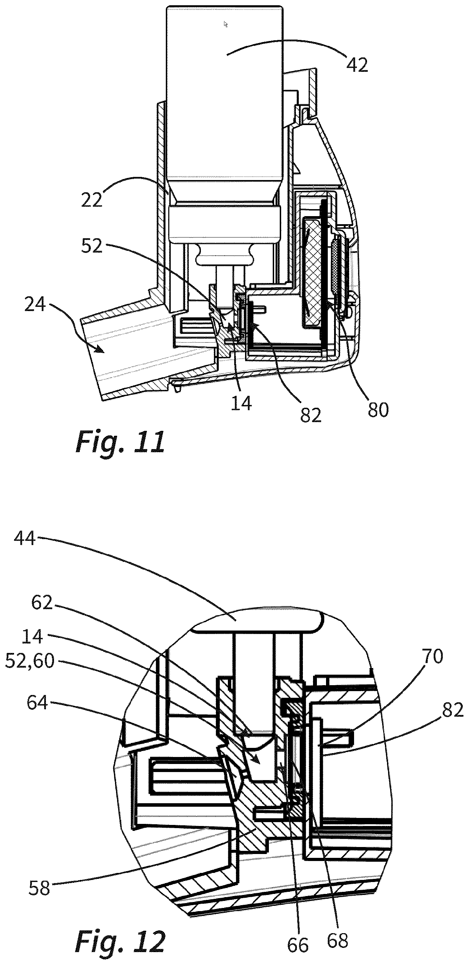

Fig. 11 zeigt den Gesamtspender in geschnittener Darstellung. Den Bereich des Kanalbauteils 58 zeigt Fig. 12 in vergrößerter Darstellung. Hier ist ersichtlich, dass das Kanalbauteil 58, welches als einstückiges Kanalbauteil ausgebildet ist, einen Kanaleingang 62 in Richtung des Aufnahmeraums 22 aufweist und am gegenüberliegenden Ende einen Kanalausgang 64 aufweist, der einen Flüssigkeitsauslass darstellt und in Richtung des Mundstücks 24 gerichtet ist. Jenseits dieses Kanalausgangs 64 vermengt sich die Flüssigkeit mit der gleichzeitig inhalierten Luft, die ein Patient einatmet, so dass ein Gemisch aus zerstäubter Flüssigkeit und Luft inhaliert wird. Der Fig. 12 ist weiterhin zu entnehmen, dass der Kanalabschnitt 60 zwischen dem Kanaleingang 62 und dem Kanalausgang 64 vollständig durch das Kanalbauteil gebildet wird und dass eine separate Messkammer 68 vorgesehen ist, die durch einen verjüngten Bereich 66 vom Kanaleingang 62 und Kanalausgang 64 getrennt ist. In dieser Messkammer erfolgt die eigentliche Messung des Flüssigkeitsaustrags, indem beim vorliegenden Ausführungsbeispiel ein membranartiger Wandungsabschnitt 70, der die Messkammer begrenzt, ausgelenkt wird. Diese Auslenkung kann auf einen Taster drücken oder mittels anderweitiger Sensoren digital oder analog erfasst werden. Die hier erfassten Daten werden von der Auswertungselektronik 80 des Auswertungsmoduls 50 verarbeitet und beispielsweise die Zahl der noch verbleibenden Dosen in der Containereinheit 40 auf dem Display 86 dargestellt. Alternativ oder zusätzlich können auch Daten zum Austrag auf einem Speicherchip der Auswertungselektronik 80 abgelegt werden und anschließend oder zu einem späteren Zeitpunkt ausgelesen werden, insbesondere über eine drahtlose Schnittstelle wie beispielsweise eine Bluetooth-Schnittstelle.

Das Auswertungsmodul 50 wird bestimmungsgemäß wiederverwendet. Es wird zu diesem Zweck teilweise vom Hauptmodul 20 entkoppelt, indem hierfür wiederum die Außenschale 54 leicht verformt wird und das Auswertungsmodul dann vom Hauptmodul abgezogen wird. Anschließend kann es an ein neues Hauptmodul 20 angekoppelt werden und dort wiederum mit einer Containereinheit 40 versehen werden. Auch ein Reinigen des Teilabschnittes 52 des Flüssigkeitskanals 14 ist nach der Trennung der Module 20, 50 sehr einfach möglich.

## Patentansprüche

1. Pharmazeutischer Spender (10), insbesondere Inhalator, zum Austrag einer pharmazeutischen Flüssigkeit, vorzugsweise in zerstäubter Form, mit den folgenden Merkmalen:
a. der Spender (10) weist einen Flüssigkeitsspeicher (42) und/oder einen Aufnahmeraum (22) zur Aufnahme eines wechselbaren Flüssigkeitsspeichers (42) auf, und
b. der Spender (10) weist einen Auslass (24) auf, durch den die Flüssigkeit aus dem Flüssigkeitsspeicher (42) in eine Umgebung abgegeben oder durch den von einem Benutzer inhaliert werden kann, und
c. der Spender (10) weist einen Flüssigkeitskanal (14) auf, der den Flüssigkeitsspeicher (42) mit dem Auslass (24) verbindet, und
d. der Spender weist eine Auswertungselektronik (80) zur elektronischen Erkennung und Verarbeitung von Austragvorgängen auf, wobei die Auswertungselektronik (80) einen Flüssigkeitssensor (82) aufweist, der zur Erfassung einer Größe ausgebildet ist, die durch das Strömen von Flüssigkeit vom Flüssigkeitsspeicher (42) zum Auslass (24) beeinflusst ist, und
e. der Spender (10) weist ein Hauptmodul (20) und ein Auswertungsmodul (50) auf, und
f. das Hauptmodul (20) umfasst den Flüssigkeitsspeicher oder den zu seiner Aufnahme vorgesehenen Aufnahmeraum (22) sowie den Auslass (24),
**gekennzeichnet durch** die folgenden Merkmale:
g. das Auswertungsmodul (50) umfasst die Auswertungselektronik (80) sowie zumindest einen Teilabschnitt (52) des Flüssigkeitskanal (14) sowie den an diesem Teilabschnitt (52) vorgesehenen Flüssigkeitssensor (82), und
h. das Hauptmodul (20) und das Auswertungsmodul (50) sind zur lösbaren Koppelung aneinander ausgebildet, und
i. das Hauptmodul (20) und das Auswertungsmodul (50) verfügen über zusammenwirkende Kopplungseinrichtungen (26,56), die zur werkzeuglosen Koppelung und Entkoppelung ausgebildet sind.

2. Pharmazeutischer Spender (10) nach Anspruch 1 mit dem folgenden weiteren Merkmal:
a. die Kopplungseinrichtungen (26,56) sind zurVerschnappung miteinander ausgebildet.

3. Pharmazeutischer Spender (10) nach Anspruch 1 oder 2 mit dem folgenden weiteren Merkmal:
a. eine äußere Oberfläche des Spenders (10) wird von dem Hauptmodul (20) und dem Auswertungsmodul (50) gemeinsam gebildet.

4. Pharmazeutischer Spender (10) nach Anspruch 3 mit den folgenden weiteren Merkmalen:
a. das Auswertungsmodul (50) weist eine Außenschale (54) auf, die im gekoppelten Zustand einen Teil der äußeren Oberfläche des Spenders (10) bildet, und
b. die Außenschale (54) weist voneinander beabstandet einen ersten und einen zweiten Teil der Kopplungseinrichtung (56) auf Seiten des Auswertungsmoduls (50) auf, wobei die beiden Teile durch elastisches Verformen der Außenschale in einen entkoppelungsfähigen Zustand gebracht werden können.

5. Pharmazeutischer Spender (10) nach einem der vorstehenden Ansprüche mit dem folgenden weiteren Merkmal:
a. der Teilabschnitt (52) des Flüssigkeitskanals (14), der Teil des Auswertungsmoduls (50) ist, wird durch einen umlaufend geschlossenen Kanalabschnitt (60) gebildet, der über einen Kanaleingang (62) und einen Kanalausgang (64) verfügt.

6. Pharmazeutischer Spender (10) nach Anspruch 5 mit dem folgenden weiteren Merkmal:
a. das Auswertungsmodul (50) weist ein einstückiges Kanalbauteil (58) auf, welches den Kanaleingang (62) und den Kanalausgang (64) bildet.

7. Pharmazeutischer Spender (10) nach Anspruch 5 oder 6 mit den folgenden weiteren Merkmalen:
a. der Flüssigkeitsspeicher (42) ist Teil einer Containereinheit (40), die zur Aufnahme im Aufnahmeraum (22) vorgesehen ist und die über einen gegenüber einer Wandung des Flüssigkeitsspeichers verlagerbaren Auslassstutzen (44) verfügt, durch den Flüssigkeit aus dem Flüssigkeitsspeicher (42) entweichen kann, und
b. der Kanaleingang (62) istzurAufnahme desAuslassstutzens (44) ausgebildet.

8. Pharmazeutischer Spender (10) nach einem der Ansprüche 5 bis 7 mit den folgenden weiteren Merkmalen:
a. der Kanalabschnitt (60) weist zwischen dem Kanaleingang (62) und dem Kanalausgang (64) einen Abzweig in eine Messkammer (68) auf, und
b. zwischen dem Abzweig und der Messkammer (68) ist eine Verjüngung (66) vorgesehen, von der aus sich der Kanalabschnitt (60) in Richtung der Messkammer und in Richtung des Abzweigs aufweitet.

9. Pharmazeutischer Spender (10) nach einem der vorstehenden Ansprüche mit den folgenden weiteren Merkmalen:
a. der Teilabschnitt (52) des Flüssigkeitskanals, der Teil des Auswertungsmoduls (50) ist, umfasst einen auslenkbaren Wandungsabschnitt (70), der in Abhängigkeit des Flüssigkeitsdrucks auslenkbar ist, und
b. dem Wandungsabschnitt (70) ist der Flüssigkeitssensor (82) in Form eines Drucksensors oder eines Schalters (82) zugeordnet, der durch die Auslenkung des Wandungsabschnitts (70) beeinflussbar oder schaltbar ist.

10. Pharmazeutischer Spender (10) nach einem der vorstehenden Ansprüche mit den folgenden weiteren Merkmalen:
a. der Teilabschnitt (52) des Flüssigkeitskanals, der Teil des Auswertungsmoduls (50) ist, umfasst den Flüssigkeitssensor, und
b. der Flüssigkeitssensor ist in einer der folgenden Arten ausgestaltet:
- als Erschütterungssensor oder als Mikrofon,
- als Temperatursensor,
- als kapazitiver Sensor oder als induktiver Sensor,
- als Differenzdrucksensor, und/oder
- als thermische Strömungssensoren.

11. Pharmazeutischer Spender (10) nach einem der vorstehenden Ansprüche mit den folgenden weiteren Merkmalen:
a. der Spender (10) weist einen Aufnahmeraum (22) zur Aufnahme eines wechselbaren Flüssigkeitsspeichers (42) auf, und
b. der Spender (10) weist einen wechselbaren Flüssigkeitsspeicher (42) auf, der Teil einer wechselbaren Containereinheit (40) ist, welche in den Aufnahmeraum (22) in einer Container-Einschubrichtung (2) eingeschoben ist, und
c. der Auslass (24) ist als Mundstück (24) ausgebildet, wobei das Mundstück (24) in einer Erstreckungsrichtung (4) ausgerichtet ist, die mit der Container-Einschubrichtung (2) einen Winkel zwischen 45° und 135° einschließt.

12. Pharmazeutischer Spender (10) nach Anspruch 11 mit dem folgenden weiteren Merkmal:
a. das Hauptmodul des Spenders bildet ein Gehäuse (28), welches auf einer dem Mundstück (24) gegenüberliegenden Seite eine Einschuböffnung (30) aufweist, in die das Auswertungsmodul (50) in einer Kopplungsrichtung (6) eingeschoben ist.

13. Pharmazeutischer Spender (10) nach einem der vorstehenden Ansprüche mit dem folgenden weiteren Merkmal:
a. das Hauptmodul (20) und das Auswertungsmodul (50) weisen eine Führungsschienenstruktur (59, 29) auf, die sich in Kopplungsrichtung (6) erstreckt,
vorzugsweise mit mindestens einem der folgenden zusätzlichen Merkmale:
b. die Kopplungsrichtung (6) ist gegenüber der Container-Einschubrichtung (2) angewinkelt, vorzugsweise in einem Winkel zwischen 60° und 120° und insbesondere vorzugsweise in einem rechten Winkel, und/oder
c. die Führungsschienenstruktur (29, 59) weist eine Führungsnut (29) auf, die an einem Ende eine Anschlagsfläche (29A) aufweist.

14. Pharmazeutischer Spender (10) nach einem der vorstehenden Ansprüche mit den folgenden weiteren Merkmalen:
a. der Spender (10) weist einen in eine Richtung offenen Aufnahmeraum (22) zur Aufnahme eines wechselbaren Flüssigkeitsspeichers (42) auf, und
b. der Spender weist eine Containereinheit (40) mit Flüssigkeitsspeicher (42) auf, die im Aufnahmeraum aufgenommen ist, wobei mindestens 50% des Flüssigkeitsspeichers (42) im Aufnahmeraum (22) aufgenommen sind.

## Claims

1. Pharmaceutical dispenser (10), in particular an inhaler, for discharging a pharmaceutical liquid, preferably in atomized form, having the following features:
a. the dispenser (10) has a liquid reservoir (42) and/or a receiving space (22) for receiving a replaceable liquid reservoir (42), and
b. the dispenser (10) has an outlet (24) through which the liquid can be dispensed from the liquid reservoir (42) into an environment or through which it can be inhaled by a user, and
c. the dispenser (10) has a liquid channel (14) which connects the liquid reservoir (42) to the outlet (24), and
d. the dispenser has an evaluation electronics unit (80) for electronic detection and processing of discharging procedures, wherein the evaluation electronics unit (80) has a liquid sensor (82) which is designed to detect a variable that is influenced by the flow of liquid from the liquid reservoir (42) to the outlet (24), and
e. the dispenser (10) has a main module (20) and an evaluation module (50), and
f. the main module (20) comprises the liquid reservoir, or the receiving space (22) provided to receive the latter, and the outlet (24), **characterized by** the following features:
g. the evaluation module (50) comprises the evaluation electronics unit (80) and at least one subportion (52) of the liquid channel (14) and also the liquid sensor (82) provided at this subportion (52), and
h. the main module (20) and the evaluation module (50) are designed for releasable coupling to each other, and
i. the main module (20) and the evaluation module (50) have interacting coupling devices (26, 56), which are designed for coupling and uncoupling without tools.

2. Pharmaceutical dispenser (10) according to Claim 1, having the following further feature:
a. the coupling devices (26, 56) are designed to be snap-fitted to each other.

3. Pharmaceutical dispenser (10) according to Claim 1 or 2, having the following further feature:
a. an outer surface of the dispenser (10) is formed by the main module (20) and the evaluation module (50) together.

4. Pharmaceutical dispenser (10) according to Claim 3, having the following further features:
a. the evaluation module (50) has an outer shell (54) which, in the coupled state, forms a part of the outer surface of the dispenser (10), and
b. the outer shell (54) has, spaced apart from each other, a first and a second part of the coupling device (56) of the evaluation module (50), wherein the two parts can be brought into an uncouplable state by elastic deformation of the outer shell.

5. Pharmaceutical dispenser (10) according to one of the preceding claims, having the following further feature:
a. the subportion (52) of the liquid channel (14), which is part of the evaluation module (50), is formed by a peripherally closed channel portion (60), which has a channel inlet (62) and a channel outlet (64).

6. Pharmaceutical dispenser (10) according to Claim 5, having the following further feature:
a. the evaluation module (50) has a one-piece channel component (58), which forms the channel inlet (62) and the channel outlet (64).

7. Pharmaceutical dispenser (10) according to Claim 5 or 6, having the following further features:
a. the liquid reservoir (42) is part of a container unit (40) provided to be received in the receiving space (22) and having an outlet nozzle (44) which is displaceable with respect to a wall of the liquid reservoir and through which liquid can escape from the liquid reservoir (42), and
b. the channel inlet (62) is designed to receive the outlet nozzle (44).

8. Pharmaceutical dispenser (10) according to one of Claims 5 to 7, having the following further features:
a. the channel portion (60) has, between the channel inlet (62) and the channel outlet (64), a branch into a measuring chamber (68), and
b. between the branch and the measuring chamber (68), a narrowing (66) is provided, from which the channel portion (60) widens in the direction of the measuring chamber and in the direction of the branch.

9. Pharmaceutical dispenser (10) according to one of the preceding claims, having the following further features:
a. the subportion (52) of the liquid channel, which is part of the evaluation module (50), comprises a deflectable wall portion (70), which is deflectable depending on the liquid pressure, and
b. the wall portion (70) is assigned the liquid sensor (82) in the form of a pressure sensor or a switch (82), which can be influenced or can be switched by the deflection of the wall portion (70) .

10. Pharmaceutical dispenser (10) according to one of the preceding claims, having the following further features:
a. the subportion (52) of the liquid channel, which is part of the evaluation module (50), comprises the liquid sensor, and
b. the liquid sensor is configured in one of the following ways:
- as a vibration sensor or as a microphone,
- as a temperature sensor,
- as a capacitive sensor or as an inductive sensor,
- as a differential pressure sensor, and/or
- as thermal flow sensors.

11. Pharmaceutical dispenser (10) according to one of the preceding claims, having the following further features:
a. the dispenser (10) has a receiving space (22) for receiving a replaceable liquid reservoir (42), and
b. the dispenser (10) has a replaceable liquid reservoir (42) which is part of a replaceable container unit (40), the latter being inserted into the receiving space (22) in a container insertion direction (2), and
c. the outlet (24) is designed as a mouthpiece (24), wherein the mouthpiece (24) is oriented in a direction of extent (4) which encloses an angle of between 45° and 135° with the container insertion direction (2).

12. Pharmaceutical dispenser (10) according to Claim 11, having the following further feature:
a. the main module of the dispenser forms a housing (28) which, on a side lying opposite the mouthpiece (24), has an insertion opening (30) into which the evaluation module (50) is inserted in a coupling direction (6).

13. Pharmaceutical dispenser (10) according to one of the preceding claims, having the following further feature:
a. the main module (20) and the evaluation module (50) have a guide rail structure (59, 29) which extends in the coupling direction (6), preferably having at least one of the following additional features:
b. the coupling direction (6) is angled with respect to the container insertion direction (2), preferably at an angle of between 60° and 120°, and particularly preferably at a right angle, and/or
c. the guide rail structure (29, 59) has a guide groove (29) which, at one end, has a stop face (29A) .

14. Pharmaceutical dispenser (10) according to one of the preceding claims, having the following further features:
a. the dispenser (10) has a receiving space (22) open in one direction for receiving a replaceable liquid reservoir (42), and
b. the dispenser has a container unit (40) with liquid reservoir (42), which is received in the receiving space, wherein at least 50% of the liquid reservoir (42) is received in the receiving space (22).

## Revendications

1. Distributeur pharmaceutique (10), notamment inhalateur, destiné à l'évacuation d'un liquide pharmaceutique, de préférence sous forme vaporisée, ayant les caractéristiques suivantes :
a. le distributeur (10) possède un réservoir à liquide (42) et/ou un espace d'accueil (22) destiné à accueillir un réservoir à liquide (42) remplaçable, et
b. le distributeur (10) possède une sortie (24) à travers laquelle le liquide peut être délivré dans l'environnement à partir du réservoir à liquide (42) ou à travers lequel il peut être inhalé par un utilisateur, et
c. le distributeur (10) possède un canal à liquide (14) qui relie le réservoir à liquide (42) à la sortie (24), et
d. le distributeur possède une électronique d'interprétation (80) destinée à la reconnaissance et au traitement électroniques d'opérations d'évacuation, l'électronique d'interprétation (80) possédant un détecteur de liquide (82) qui est conçu pour acquérir une grandeur, laquelle est influencée par l'écoulement du liquide du réservoir à liquide (42) vers la sortie (24), et
e. le distributeur (10) possède un module principal (20) et un module d'interprétation (50), et
f. le module principal (20) comporte le réservoir à liquide ou l'espace d'accueil (22) destiné à accueillir celui-ci ainsi que la sortie (24),
**caractérisé par** les caractéristiques suivantes :
g. le module d'interprétation (50) comporte l'électronique d'interprétation (80) ainsi qu'au moins une portion partielle (52) du canal à liquide (14) ainsi que le détecteur de liquide (82) présent au niveau de cette portion partielle (52), et
h. le module principal (20) et le module d'interprétation (50) sont configurés pour un accouplement amovible l'un à l'autre, et
i. le module principal (20) et le module d'interprétation (50) disposent de dispositifs d'accouplement (26, 56) coopérants qui sont configurés pour l'accouplement et le désaccouplement sans outil.

2. Distributeur pharmaceutique (10) selon la revendication 1, comprenant la caractéristique supplémentaire suivante :
a. les dispositifs d'accouplement (26, 56) sont configurés pour s'encliqueter l'un avec l'autre.

3. Distributeur pharmaceutique (10) selon la revendication 1 ou 2, comprenant la caractéristique supplémentaire suivante :
a. une surface extérieure du distributeur (10) est formée communément par le module principal (20) et le module d'interprétation (50).

4. Distributeur pharmaceutique (10) selon la revendication 3, comprenant les caractéristiques supplémentaires suivantes :
a. le module d'interprétation (50) possède une coque externe (54) qui, à l'état accouplé, forme une partie de la surface extérieure du distributeur (10), et
b. la coque externe (54) possède une première et une deuxième partie du dispositif d'accouplement (56), espacées l'une de l'autre, sur les côtés du module d'interprétation (50), les deux parties étant amenées dans un état apte au désaccouplement par déformation élastique de la coque externe.

5. Distributeur pharmaceutique (10) selon l'une des revendications précédentes, comprenant la caractéristique supplémentaire suivante :
a. la portion partielle (52) du canal à liquide (14) qui fait partie du module d'interprétation (50) est formée par une portion de canal (60) fermée en circonférence, laquelle dispose d'une entrée de canal (62) et d'une sortie de canal (64).

6. Distributeur pharmaceutique (10) selon la revendication 5, comprenant la caractéristique supplémentaire suivante :
a. le module d'interprétation (50) possède un composant de canal monobloc (58) qui forme l'entrée de canal (62) et la sortie de canal (64).

7. Distributeur pharmaceutique (10) selon la revendication 5 ou 6, comprenant la caractéristique supplémentaire suivante :
a. le réservoir à liquide (42) est une partie d'une unité de contenant (40), laquelle est conçue pour être accueillie dans l'espace d'accueil (22) et laquelle dispose d'un manchon de sortie (44) positionnable par rapport à une paroi du réservoir à liquide, à travers lequel le liquide peut s'échapper hors du réservoir à liquide (42), et
b. l'entrée de canal (62) est configurée pour accueillir le manchon de sortie (44).

8. Distributeur pharmaceutique (10) selon l'une des revendications 5 à 7, comprenant les caractéristiques supplémentaires suivantes :
a. la portion de canal (60) possède, entre l'entrée de canal (62) et la sortie de canal (64), une bifurcation dans une chambre de mesure (68), et
b. un rétrécissement (66) se trouve entre la bifurcation et la chambre de mesure (68), à partir duquel la portion de canal (60) s'élargit en direction de la chambre de mesure et en direction de la bifurcation.

9. Distributeur pharmaceutique (10) selon l'une des revendications précédentes, comprenant les caractéristiques supplémentaires suivantes :
a. la portion partielle (52) du canal à liquide qui fait partie du module d'interprétation (50) comporte une portion de paroi (70) pouvant être déviée, laquelle peut être déviée en fonction de la pression du liquide, et
b. le détecteur de liquide (82) est associé à la portion de paroi (70) sous la forme d'un capteur de pression ou d'un commutateur (82) qui peut être influencé ou peut être commuté par la déviation de la portion de paroi (70).

10. Distributeur pharmaceutique (10) selon l'une des revendications précédentes, comprenant les caractéristiques supplémentaires suivantes :
a. la portion partielle (52) du canal à liquide qui fait partie du module d'interprétation (50) comporte le détecteur de liquide, et
b. le détecteur de liquide est réalisé de l'une des manières suivantes :
- sous la forme d'un détecteur de secousses ou d'un microphone,
- sous la forme d'une sonde de température,
- sous la forme d'un capteur capacitif ou d'un capteur inductif,
- sous la forme d'un capteur de pression différentielle et/ou
- sous la forme de détecteurs de débit thermiques.

11. Distributeur pharmaceutique (10) selon l'une des revendications précédentes, comprenant les caractéristiques supplémentaires suivantes :
a. le distributeur (10) possède un espace d'accueil (22) destiné à accueillir un réservoir à liquide (42) remplaçable, et
b. le distributeur (10) possède un réservoir à liquide (42) remplaçable, qui est une partie d'une unité de contenant (40) remplaçable dans laquelle l'espace d'accueil (22) est inséré dans une direction d'insertion de contenant (2), et
c. la sortie (24) est réalisée sous la forme d'un embout buccal (24), l'embout buccal (24) étant orienté dans une direction d'extension (4) qui forme avec la direction d'insertion de contenant (2) un angle entre 45° et 135°.

12. Distributeur pharmaceutique (10) selon la revendication 11, comprenant la caractéristique supplémentaire suivante :
a. le module principal du distributeur forme un boîtier (28), lequel possède sur le côté opposé à l'embout buccal (24) une ouverture d'insertion (30) dans laquelle est inséré le module d'interprétation (50) dans une direction d'accouplement (6).

13. Distributeur pharmaceutique (10) selon l'une des revendications précédentes, comprenant la caractéristique supplémentaire suivante :
a. le module principal (20) et le module d'interprétation (50) possèdent une structure de rail de guidage (59, 29) qui s'étend dans la direction d'accouplement (6),
comprenant de préférence au moins l'une des caractéristiques supplémentaires suivantes :
b. la direction d'accouplement (6) forme un angle par rapport à la direction d'insertion de contenant (2), de préférence un angle entre 60° et 120°, et notamment un angle droit, et/ou
c. la structure de rail de guidage (29, 59) possède une rainure de guidage (29) qui possède une surface de butée (29A) à une extrémité.

14. Distributeur pharmaceutique (10) selon l'une des revendications précédentes, comprenant les caractéristiques supplémentaires suivantes :
a. le distributeur (10) possède un espace d'accueil (22) ouvert dans une direction destiné à accueillir un réservoir à liquide (42) remplaçable, et
b. le distributeur possède une unité de contenant (40) comprenant un réservoir à liquide (42), laquelle est accueillie dans l'espace d'accueil, au moins 50 % du réservoir à liquide (42) étant accueillis dans l'espace d'accueil (22).
